# EUROPÄISCHE PATENTANMELDUNG

(11) **EP 1 645 267 A2**
(43) Veröffentlichungstag der Anmeldung: **12.04.2006**
(21) Anmeldenummer: 05027278.0
(22) Anmeldetag: 11.02.2002
(51) Int. Cl.: A61K 9/107, A23L 1/30, A61K 31/20, A61K 31/355, A61K 31/381, A61K 31/575

(54) **Verfahren zur Herstellung eines Wirkstoffkonzentrats sowie Wirkstoffkonzentrat**

(30) Priorität: 11.02.2001 DE 10109708; 22.02.2001 DE 10108614
(62) Teilanmeldung aus: 02719799.5
(71) Anmelder: AQUANOVA German Solubilisate Technologies (AGT) GmbH, 64295 Darmstadt (DE)
(72) Erfinder: Behnam, Dariush, 64380 Rossdorf (DE)
(74) Vertreter: Blumbach - Zinngrebe

(57) **Zusammenfassung**

Beschrieben wird ein Verfahren zur Herstellung einer wasserlöslichen Phase eines in Wasser nicht oder nur schwer löslichen physiologisch wirksamen Wirkstoffes, bei dem der eine ω-3-Fettsäure enthaltende Wirkstoff in der Wärme mit einem körperverträglichen Lösungsvermittler mit einem HLB-Wert zwischen 9 und 16 im Überschuss zur Bildung eines transparenten Zwischenproduktes verrührt wird und das Zwischenprodukt auf Raumtemperatur abgekühlt wird. Ferner wird ein wasserlösliches Konzentrat eines physiologischen, eine ω-3-Fettsäure enthaltenden Wirkstoffes erläutert.

## Beschreibung

Die Erfindung betrifft ein wasserlösliches Konzentrat, welches einen physiologischen Wirkstoff, der in Wasser unlöslich oder nur schwer löslich ist, sowie einen Lösungsvermittler aufweist, sowie Verfahren zur Herstellung der Konzentrate.

Fettlösliche Verbindungen wie z.B. Vitamin E, Vitamin A und andere Carotinoide oder auch Coenzym Q₁₀ werden in Abhängigkeit vom Vorhandensein von Gallensalzen und Enzymen der Bauchspeicheldrüse absorbiert. Dem Absorptionsprozess geht ein Vorgang der sogenannten Micellenbildung im Darm voraus, der erforderlich ist, damit die fettlöslichen Verbindungen "verpackt" werden und auf diese Weise verschiedene Barrieren der Darmmucosa überwinden können.

Ist die Sekretion von Gallenflüssigkeit oder Enzymen der Bauchspeicheldrüse gestört, so resultiert daraus eine sogenannte Maldigestion oder Malabsorption fettlöslicher Verbindungen. Das beste Beispiel hierfür ist der Krankheitszustand der Zystischen Fibrose, bei der aufgrund mangelnder Bereitstellung von Bauchspeicheldrüsenenzymen die Resorption fettlöslicher Verbindungen nur noch in sehr geringem Umfang möglich ist.

Die Besonderheiten der Absorption fettlöslicher Mikronährstoffe zeigt sich auch daran, dass die Aufnahme immer dann steigt, wenn gleichzeitig Fett angeboten wird. Fett begünstigt einerseits die Abgabe von Gallensäure und Enzymen der Bauspeicheldrüse und andererseits die Bildung von Micellen, die dann die besagten fettlöslichen Mikronährstoffe enthalten.

Nachdem die fettlöslichen Verbindungen von den Darmzellen aufgenommen worden sind, liegen sie dort in freier Form vor, d.h. sie sind nicht mehr an micellare Bestandteile gebunden. In dieser freien Form werden sie dann erneut "wasserlöslich" gemacht, indem sie in innerhalb der Darmzellen gebildete Transporter eingebaut (Lipoproteine - Chylomikronen) und dann über die großen Lymphwege ins Blut abgegeben werden.

Um lipophile Verbindungen aufnehmen zu können, muss sie der Organismus also in zwei Schritten wasserlöslich machen. Der erste Schritt erfolgt im Darm durch die Bildung der Micellen, aus denen dann die Substanz in der Darmzelle wieder freigesetzt wird, und der zweite Schritt ist die Bildung von Lipoproteinen zum Transport im Blut. Daher werden lipophile Substanzen, die wasserlöslich gemacht worden sind (klare Lösungen), nicht aber solche, die im wässrigen Medium lediglich dispergiert sind (trübe Lösungen), vom Organismus rascher und effizienter absorbiert als die ursprünglich lipophile Substanz.

Über die Bioverfügbarkeit wasserlöslich gemachter lipophiler Mikronährstoffe (klare Lösungen) liegen nur wenig Daten vor. Ein Verfahren zur Prüfung der Bioverfügbarkeit solcher Verbindungen sind sogenannte in-vitro-Dissolutionsverfahren. Hierbei wird festgestellt, inwieweit sich eine Verbindung im wässrigen Kompartiment löst bzw. inwieweit sie aus einer bestimmten galenischen Zubereitungsform freigesetzt wird. Aus US-6,048,566 ist bekannt, dass wasserlöslich gemachtes Q₁₀ im Gegensatz zu Q₁₀ aus öligen Lösungen oder Dispersionen (trübe Lösungen) zu 100 Prozent freigesetzt wird. Dies bedeutet aber, dass das so applizierte Q₁₀ bereits in höherer Konzentration in freier Form im Darmlumen vorliegt und auf diese Weise nicht erst durch Micellenbildung oder durch Abbau der das Q₁₀ umgebenden Lipide freigesetzt werden muss.

Der Erfindung liegt deshalb die Aufgabe zugrunde, die Bioverfügbarkeit von ursprünglich in Wasser nicht oder nur schwer löslichen, physiologisch bedeutsamen Substanzen wie ω-3-Fettsäuren, α-Liponsäure (Thioctsäure), Ubichinonen (z.B. Coenzym Q₁₀), Phytosterinen und anderen zu verbessern und die industrielle Verarbeitung dieser Substanzen technologisch zu erleichtern.

Der Erfindung liegt auch die Aufgabe zugrunde, bei der Herstellung der Konzentrate die kleinstmögliche Menge an Lösungsvermittlern also insbesondere Polysorbaten - unter Berücksichtigung eines Toleranzbereichs zur Sicherung der vollständigen, stabilen Wasserlöslichkeit - einzusetzen, so dass die ADI-Werte (ADI=Acceptable Daily Intake) für die Polysorbate gemäß JECFA (=Joint FAO/WHO Expert Committee on Food Additives) und SCG-Substanzen (SCG = Scientific Committee on Food (EU)) wie Tocopherolen (z.B. α-Tocopherolen), ω-3-Fettsäuren, α-Liponsäure (Thioctsäure) und Ubichinonen (z.B. Coenzym Q₁₀) deutlich unterschritten werden.

Zur Erfüllung dieser Aufgaben ist erfindungsgemäß vorgesehen, dass der in Wasser nicht oder nur schwer lösliche Wirkstoff in einen Überschuß an erwärmtem körperverträglichen Lösungsvermittler mit einem HLB-Wert zwischen 6 und 19 , insbesondere Polysorbat 20 oder Polysorbat 80, eingetragen, die Mischung solange in der Wärme gerührt wird, bis sich ein klares zähflüssiges Zwischenprodukt ergibt, und das Zwischenprodukt anschließend auf Zimmertemperatur abgekühlt wird. Soll der Wirkstoff in wässriger Phase bereitgestellt werden, wird dem warmen Zwischenprodukt warmes destilliertes Wasser in solcher Menge beigegeben, wie der erwünschten Wirkstoffkonzentration entspricht, bis zur Homogenität gerührt und die so entstandene Phase rasch auf Zimmertemperatur abgekühlt.

Soll die Phase wasserfrei sein, wird dem warmen Zwischenprodukt ein warmes Triglycerid, beispielsweise ein leichtes Pflanzenöl mit hohem Linolsäuregehalt sowie warmes Polysorbat in solcher Menge zugegeben, daß sich die gewünschte Wirkstoffkonzentration ergibt, und erneut in der Wärme bis zur Klarheit des Konzentrats gerührt. Nach Abkühlen liegt der Wirkstoff in wasserfreier, jedoch in Wasser beliebig löslicher Phase vor. Diese eignet sich besonders zur Darreichung des Wirkstoffes in Kapselform, welche nur einen sehr geringen Wassergehalt auf Dauer toleriert.

Sowohl die wasserfreie wie die wässrige Phase sind in Wasser bzw. Fett und/oder Öl löslich.

Wie Messungen mittels einphasiger Chromatographie zeigen, liegt der Wirkstoff sowohl in der wässrigen wie in der wasserfreien Phase in von Polysorbat eingehüllten Molekülaggregaten vor, wobei die Polysorbathülle jeweils einen Durchmesserbereich von etwa 30 nm aufweist, die Polysorbathülle mit eingeschlossenem Molekülaggregat also als eine Micelle anzusehen ist.

Im physiologischen Bereich hat die erfindungsgemäße Micellenbildung eine wesentlich verbesserte Bioverfügbarkeit des Wirkstoffes zur Folge. Der in Wasser nicht oder nur schwer lösliche Wirkstoff braucht nicht erst durch Einwirkung von Gallensekreten für das Darmlumen aufnahmefähig gemacht zu werden.

Ausführungsbeispiele der Erfindung sind in den Unteransprüchen angegeben und nachstehend ohne Beschränkung der Allgemeinheit der beanspruchten Erfindung erläutert. In der beigefügten Zeichnung zeigen
Figur 1 eine Darstellung gemessener Radiusverteilungen von Micellen;
Figur 2 eine schematische Erläuterung von Wirkstoffinicellen am Beispiel des Coenzyms Q₁₀ und
Figur 3 eine schematische Erläuterung der Anordnung von Micellen, die einen Wirkstoff (Coenzym Q₁₀) sowie einen Hilfsstoff (Linolsäure) enthalten;
Figur 4: eine schematische Darstellung von Phytosterinmicellen;
Figur 5: eine schematische Darstellung der Verteilung zusätzlicher Linolsäuremicellen um eine Phytosterinmicelle, und
Figur 6: eine schematische Darstellung einer Micelle einer ω 3 Fettsäure.

### Beispiel 1: Coenzym Q₁₀

In 77 Massenteile von auf 85°C erwärmtes Polysorbat 80 werden 23 Massenteile Coenzym Q₁₀ eingetragen und die Mischung wird etwa 5 min bei 85°C gerührt, bis sich eine klare zähflüssige Masse leicht gelblicher Farbe ergibt. Die Massenanteile im Zwischenprodukt von Coenzym Q₁₀ zu Polysorbat 80 betragen 1 : 3,35 und das Verhältnis der Molekülzahlen beträgt 1 : 2,56, da das Molekulargewicht von Coenzym Q₁₀ 863,36 und dasjenige von Polysorbar 80 1130,00 beträgt.

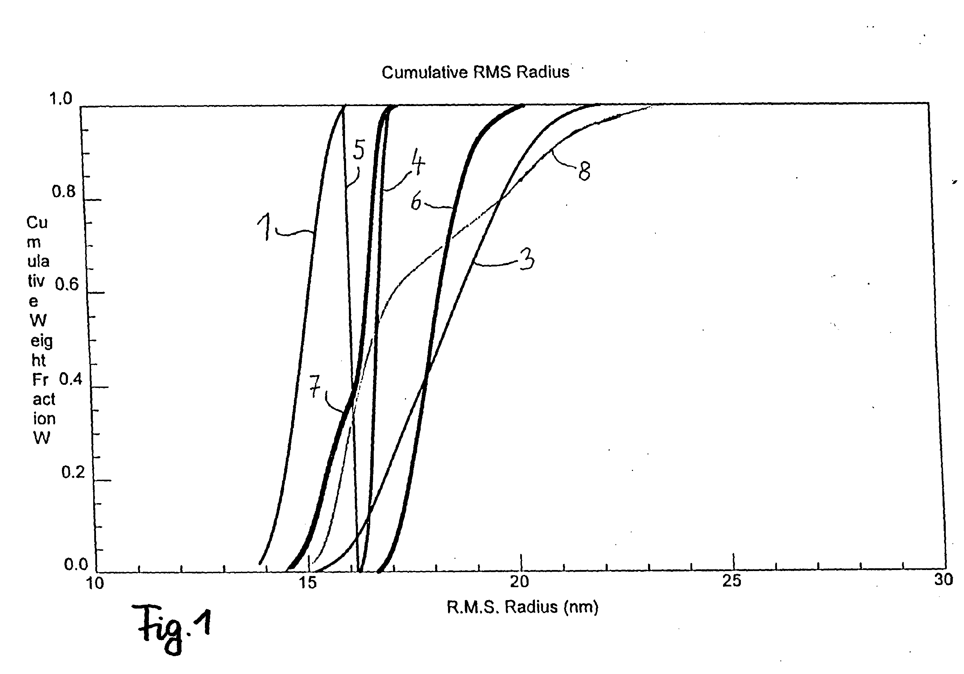

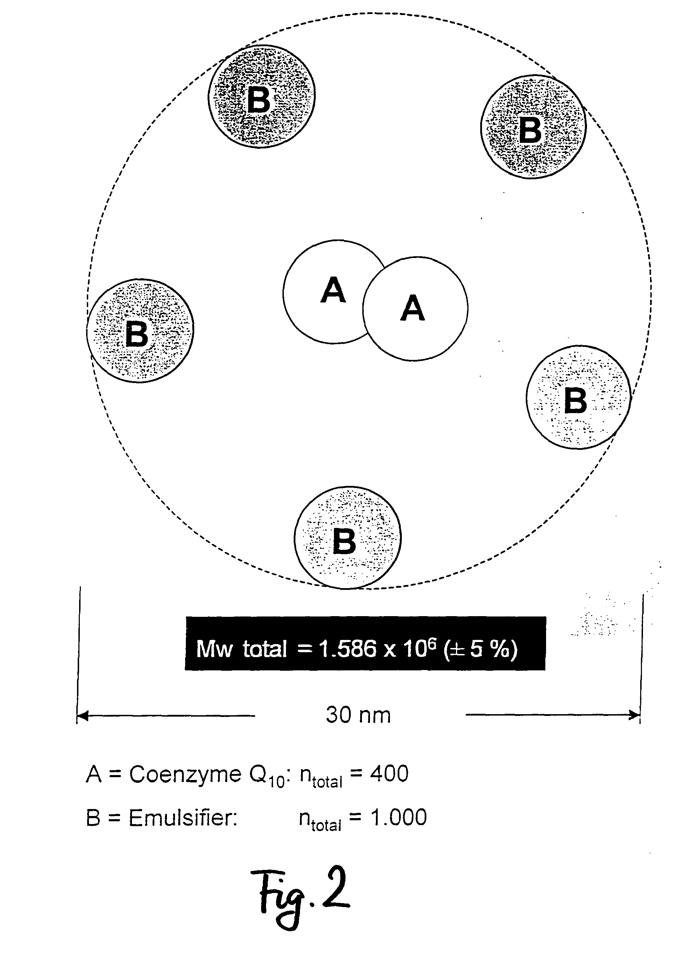

Zur Gewinnung einer wässrigen Phase von Q₁₀ aus dem Zwischenprodukt, welche eine Q₁₀ Konzentration von etwa 3% enthält, wird dem warmen Zwischenprodukt etwa 865 Gewichtsteile an warmem Wasser zugesetzt und in der Wärme erneut gerührt, bis sich eine klare Flüssigkeit ergibt. Anschließend wird die Flüssigkeit rasch (beispielsweise innerhalb etwa 1 bis 2 Minuten) auf Zimmertemperatur (etwa 20°C) zur fertigen wässrigen Phase des Coenzyms Q₁₀ heruntergekühlt. Der durchschnittliche in der Phase vorliegende Partikelradius wurde durch Feld-Fluß-Fraktionierung (FFF) mit an die Chromatographiesäule angekoppeltem DAWN EOS-Detektor der Firma Wyatt Technologie Deutschland GmbH erfaßt. Wie die Kurve 1 in Figur 1 zeigt, liegt der Radius in Abhängigkeit von der kumulativen Gewichtsfraktion zwischen etwa 14 nm und etwa 16 nm und damit im Größenbereich der Micellen. Aus dem hierbei bestimmten durchschnittlichen Micellengewicht von etwa 1,586 x 10⁶ Einheiten läßt sich errechnen, daß jede Micelle im Kern zwei Molekülaggregate von insgesamt etwa 400 Molekülen Coenzym Q₁₀ aufweist, welcher von fünf untereinander gleichartigen Molekülaggregaten Polysorbat 80 von insgesamt etwa 1000 Molekülen Polysorbat 80 umgeben ist, wie schematisch in Figur 2 dargestellt ist.

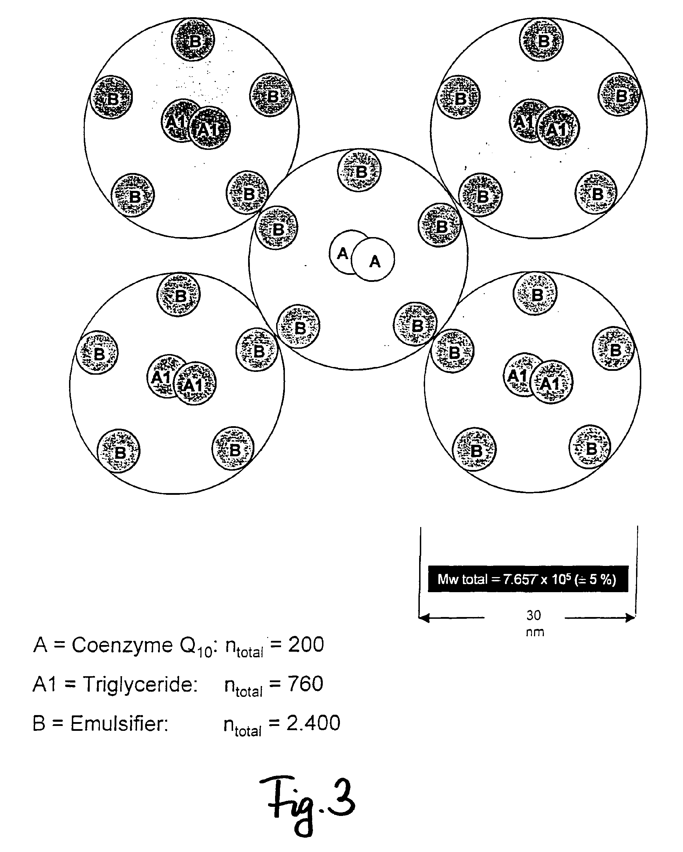

Zur Gewinnung der wasserfreien Phase von 5 Gew% Coenzym Q₁₀ werden dem warmen Zwischenprodukt soviel Triglycerid und Polysorbat 80 in der Wärme (85°C) zugesetzt, daß die Mischung etwa 5 Teile Coenzym Q₁₀ , etwa 16 Teile Triglycerid und etwa 79 Teile Polysorbat 80 aufweist. Als Triglycerid wird hier Distelöl eingesetzt, welches nach den Leitsätzen für Speisefette und Speiseöle vom 29/30.11.1983 in der am 2./3.12.1986 geänderten Fassung (GMB1. Nr. 21, Seite 379 vom 1.8.1987) einen hohen Linolsäuregehalt, nämlich von etwa 67,8% bis etwa 83,2%, aufweist. Linolsäure empfiehlt sich wegen seiner dem Coenzym Q₁₀ ähnlichen Molekülgröße (Molekulargewicht der Linolsäure 725). Die Mischung wird bis zur Klarheit in der Wärme gerührt und alsdann langsam abgekühlt. Man erhält eine wasserfreie Phase von Coenzym Q₁₀ , deren Partikelgröße aufgrund der genannten Messungen ebenfalls im Micellenbereich liegt. Im Unterschied zur wässrigen Phase wird mit der genannten Methode ein Micellendurchmesseer von durchschnittlich 7,657 x 10⁵ gemessen aus welchem sich ergibt, daß jede Micelle im Kern etwa 200 Coenzym Q₁₀ Moleküle und fünf umlagernde Molekülaggregate von insgesamt etwa 480 Molekülen Polysorbat 80 aufweist, wobei diese Micelle von vier weiteren untereinander gleichartigen Micellen umgeben ist, von denen jede im Kern etwa 190 Moleküle Distelöl bezw. Linolsäure und eine Polysorbathülle aus fünf Molukülaggregaten von insgesamt etwa 480 Molekülen Polysorbat aufweist. Eine schematische Darstellung dieser Micellenausbildung zeigt Figur 3.

Die wasserfreie Phase ist ausgezeichnet lagerfähig und läßt sich in Wasser bei Körpertemperatur beliebig lösen. Sie ist deshalb als Zusatz zu Nahrungsergänzungsmitteln geeignet, die üblicherweise in Gelatinekapseln angeboten werden. Eine Erklärung für die besondere Beständigkeit der wasserfreien Phase kann unter Umständen darin gesehen werden, daß die zentrale, das Coenzym Q₁₀ enthaltende Micelle durch die vier umgebenden, den Hilfsstoff Distelöl d.h. im wesentlichen also Linolsäure enthaltenden Micellen vor dem Eindringen insbesondere polarer Moleküle wie H₂O weitgehend geschützt ist.

### Beispiel 2: Phytosterin

Ausgegangen wird von dem ADM Phytosterol, das unter dem Produktcode 040095 von der ADM Nutraceutical, Decatur, Illinois 62526, U.S.A. bezogen werden kann. Dieses Produkt enthält wenigstens 90% Phytosterine, und zwar 40% - 58% Beta_Sitosterin, 20% - 30% Campesterin und 14% - 22% Stigmasterin sowie je bis zu etwa 5% Sitostanin und Brassicasterin. Nachfolgend wird dieses Produkt kurz als Phytosterin bezeichnet. Selbstverständlich lassen sich Phytosterine auch anderer Hersteller, die andere Zusammensetzungen in anderer Konzentration enthalten, wie nachstehend beschrieben mit den entsprechenden Ergebnissen behandeln. Die Erfindung ist daher auf das ADM Phytosterol nicht beschränkt.

Zur Herstellung einer wässrigen Phase werden etwa 320 g Polysorbat, vorzugsweise Polysorbat 80, auf etwa 100°C erhitzt. Dem warmen Polysorbat werden etwa 10 g Phytosterin hinzugegeben und die Mischung wird unter Beibehaltung der Temperatur von etwa 100°C solange, das heißt etwa 10 Minuten, gerührt, bis sich eine homogene und transparente etwa 3%ige Phytosterinkonzentration ergibt. Die entstehende wasserfreie Phase ist, gegebenenfalls nach Erwärmen auf etwa 40°C, beliebig in Wasser von 20°C löslich.

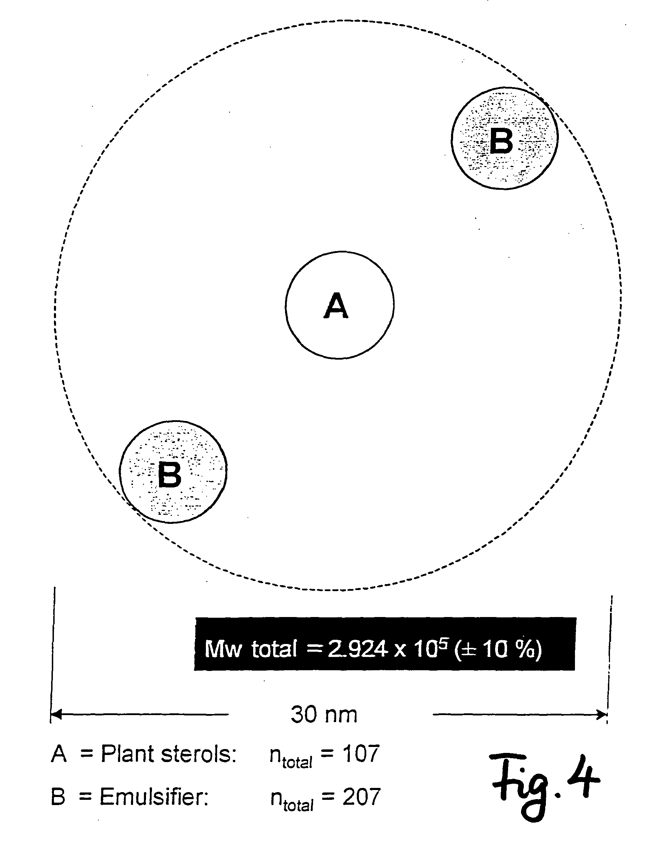

Die genannte Untersuchung zeigt, daß Micellen mit einer Molekülaggregatanordnung gemäß Figur 4 vorliegen, wobei im Micellenkern etwa 107 Phytosterin-"Moleküle" und in der Polysorbat 80 - Hülle etwa 207 Polysorbatmoleküle vorhanden sind. Die Radienverteilung der Micellen ist aus Kurve 3 der Figur 1 ersichtlich und liegt zwischen etwa 15 nm und etwa 22 nm.

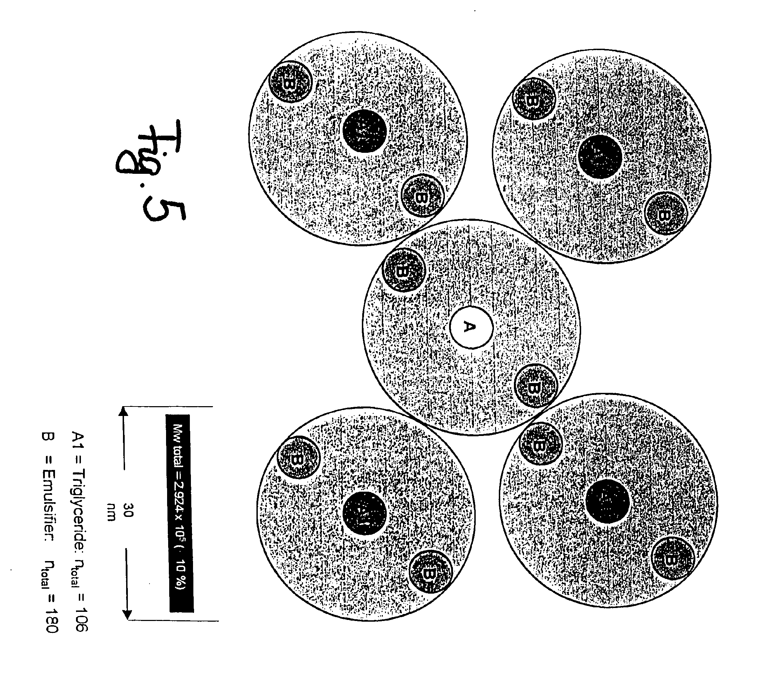

Zur Herstellung einer wasserfreien Phase werden zunächst etwa 100 g leichtes Pflanzenöl, beispielsweise Distelöl, auf etwa 100°C erhitzt. Zu dem heißen Distelöl werden etwa 10 g Phytosterin gegeben und die Mischung unter Beibehaltung der Temperatur von etwa 100°C solange, beispielsweise 10 Minuten, gerührt, bis sich das Phytosterin vollständig gelöst hat. Dieser Lösung werden etwa 220 g Polysorbat, vorzugsweise Polysorbat 80, zugegeben. Das Rühren wird bei etwa 100°C solange fortgesetzt, bis die Mischung transparent von leicht gelblicher Farbe geworden ist. Nach Abkühlen auf Zimmertemperatur liegt die wasserfreie Phase einer etwa 3%igen Phytosterinkonzentration vor. Sie ist in Wasser und Ölen löslich und bleibt gegenüber Salz- oder Magensäure (pH < 1) und auch gegenüber Wärmeeinwirkung stabil. Messungen zeigen, daß etwa 90% der Partikel der wasserfreien Phase einen Radius um die 16 nm zeigen. Die Molekülaggregatanordnung zeigt schematisch Figur 5, gemäß welcher die Phytosterinmicelle von vier Linolsäuremicellen umgeben ist.

Die cholesterinsenkende Wirkung der Phytosterine ist seit Jahrzehnten bekannt und durch neuere Studien klinisch belegt.

### Beispiel 3: ω 3 Fettsäure

Als Beispiel für eine ω-3-Fettsäure-wird das Produkt Softgel verwendet, das von Merck KGaA, Darmstadt, bezogen werden kann und dort unter der Produktnummer 1.00743.0200 HI-DHA 25 S geführt wird. Dieses Produkt enthält 25% - 28% Decosahexaensäure (DHA), 5% - 8% Eicosapentaensäure (EPA) und insgesamt etwa 34% - 40% ω 3 Fettsäure. Nachfolgend wird dieses Produkt kurz als Omega-3-Fettsäure bezeichnet. Selbstverständlich können auch ω 3 Fettsäuren anderer Hersteller wie nachfolgend beschrieben behandelt werden, wobei sich analoge Ergebnisse einstellen.

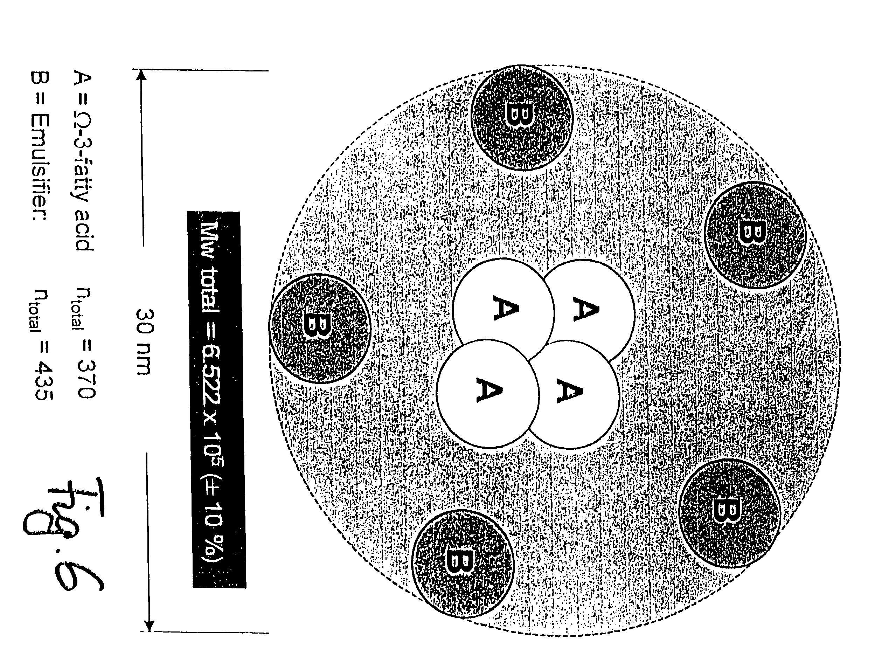

Als Beispiel für die Herstellung eines ω-3-Fettsäure-Konzentras werden etwa 800 Gramm Polysorbat 80 auf etwa 160 Grad Celsius erhitzt. Anschließend werden unter Beibehaltung der Temperatur etwa 200 Gramm Omega-3-Fettsäure hinzugegeben und unter Beibehaltung der Temperatur etwa 5 Minuten lang gerührt, bis sich eine homogene Mischung als Zwischenprodukt ergeben hat. Das so hergestellte Zwischenprodukt ist transparent und behält seine Transparenz auch nach langsamer Abkühlung auf Zimmertemperatur bei. Es ist in etwa 20°C warmem Wasser nach kurzem Rühren beliebig löslich, ohne daß Trübungen oder eine Sedimentierung eintreten. Das Zwischenprodukt, das etwa 6% ω 3 Fettsäuren enthält, weist Micellen auf, deren durchschnittliche Radiusverteilung in Kurve 4 der Figur 1 angegeben ist, wie Messungen des wässrigen Zwischenproduktes nach der vorstehend angegebenen Methode ergeben haben. Der durchschnittliche Micellendurchmesser liegt also bei etwa 33 nm. Die Molekülaggregatanordnung zeigt Figur 6, auf die dort eingetragen Zahlenwerte wird Bezug genommen.

Klarheit und Wasserlöslichkeit des wässrigen Zwischenproduktes bleiben auch dann erhalten, wenn diesem Magensäure (Salzsäure) zugegeben wird.

Ein Kilogramm des Zwischenproduktes enthält etwa 67 Gramm ω-3-Fettsäuren, so daß etwa 3 bis 4 Gramm dieses Zwischenproduktes deckt den menschlichen Tagesbedarf an ω-3-Fettsäuren decken.

### Beispiel 4: Isoflavone

Ausgegangen wird von einem Sojabohnenextraktpulver, das von der Archer-Daniels-Midland Company, U.S.A. unter der Marke NOVASOY bezogen werden kann. Dieses Produkt enthält mindestens 40 Gew% Genistin, Daidcin und Glycitin und ihrer Aglykone im Mengenverhältnis 1,3:1,0:0,3. Daher enthalten 100g des genannten Extraktes 20,0g Genistin, 15,4g Daidcin und 4,6g Glycitin, also insgesamt 40g Isoflavone. Nachfolgend wird dieses Produkt kurz als Genistin-Isoflavon bezeichnet. Selbstverständlich kann auch ein entsprechendes Produkt eines anderen Herstellers eingesetzt werden, sofern es Isoflavone gegebenenfalls in anderer Zusammensetzung enthält. So ist beispielsweise von der K.-W. Pfannenschmidt GmbH, Hamburg ein Sojabohnenextrakt erhältlich, das etwa 7,58% Genistin, 25,43% Genistein, 5,48% Daidcin und 1,67% Daidcein, also etwa 40% Isoflavone enthält. Nach der nachstehend beschriebenen Behandlung lassen sich etwa die gleichen Ergebnisse erzielen.

Etwa 166g des Genistin-Isoflavons werden in etwa 834g auf etwa 75°C erwärmtes Polysorbat 80 eingerieselt und die Mischung bei dieser Temperatur etwa eine halbe Stunde lang gleichmäßig gerührt. Es ergibt sich ein klares, tiefbraunes Zwischenprodukt ohne Sediment. Gibt man etwa 1―2 ml dieses Zwischenproduktes zu der zehnfachen Menge an destilliertem Wasser von Zimmertemperatur, erhält man eine klare wässrige Phase. Wie die Messungen zeigen, liegen in ihr Micellen vor, von denen etwa 96,1% einen Radius von etwa 16nm bis etwa 20nm besitzen, wie Kurve 5 in Figur 1 zeigt.

Nach einer alternativen Verfahrensführung werden etwa 100g des Genistin-Isoflavons gleichmäßig in etwa 400g Wasser eingerührt, das zuvor auf etwa 60°C erwärmt wurde. Die Mischung wird unter Beibehaltung der Temperatur etwa 10 Minuten gerührt und anschließend unter Fortsetzung des Rührvorgangs etwa 500g Polysorbat 80 hinzugegeben und während der Zugabe die Temperatur auf etwa 100°C erhöht. Bei dieser Temperatur wird der Rührvorgang solange fortgesetzt, bis sich ein klares Zwischenprodukt ergeben hat.

### Beispiel 5: Quercetin

Für ein Quercetin enthaltendes Mittel kann man von einem Quercetin-Dihydrat ausgehen, das von der Sigma_Aldrich-Chemie GmbH, Schnelldorf unter der Artikelnummer 83370-100G bezogen werden kann.

67g Quercetin-Dihydrat werden gleichmäßig in etwa 280g Wasser eingerührt, das zuvor auf etwa 60°C erwärmt wurde. Die Mischung wird unter Beibehaltung der Temperatur etwa 5 Minuten konstant (etwa durch einen Magnetrührer) gerührt und anschließend während des Rührens etwa 653g Polysorbat 80 hinzugegeben, wobei die Temperatur auf ungefähr 100°C erhöht wird. Das Rühren wird solange fortgesetzt, bis sich ein klares, transparentes Zwischenprodukt mit einem Gehalt von etwa 6,7% Quercetin ergibt. Eine wässrige Lösung desselben zeigt Micellen, von denen etwa 90% eine Radiusverteilung zwischen 17nm und 19nm aufweisen (Kurve 6 in Figur 1).

### Beispiel 6: Lycopin

Ausgegangen wird von einem Produkt, das von BASE S.A., Schweiz, unter dem Namen Tomato Oleoresin bezogen werden kann. Es enthält etwa 40% Lycopin und wird nachfolgend kurz Base-Lycopin genannt. Ein Lycopin enthaltendes Produkt kann auch von der LycoRed Natural Products Industries Ltd, Beer-Sheva, Israel bezogen werden.

Etwa 100g Wasser werden auf knapp 100°C erhitzt und etwa 50g Base-Lycopin dem heißen Wasser zugegeben. Unter Beibehaltung der Temperatur wird etwa 5 Minuten kräftig gerührt, bis die Mischung homogen und transparent geworden ist. Anschließend werden etwa 850g Polysorbat 80 auf etwa 100°C erhitzt und der Mischung zugegeben. Das entstandene Gesamtgemisch wird solange bei etwa 100°C gerührt, bis sich ein homogenes und transparentes Zwischenprodukt ergibt. Nach Abkühlung auf Raum- oder Körpertemperatur bleiben Klarheit und Wasserlöslichkeit der erhaltenen wässrigen Phase mit einem 2%igen Lycopingehalt erhalten. Gemäß Kurve 7 von Figur 1 haben etwa 86% der entstandenen Micellen einen Radius von etwa 15nm bis etwa 16nm.

Kurve 8 in Figur 1 bezieht sich auf reines Polysorbat 80 ohne Wirkstoffe

### Beispiel 7: Chondroitinsulfat

Das polymere Galactosaminsulfat Chondroitin unterstützt die Regeneration von überbelastetem Knorpelgewebe und reduziert Symptome von Osteoarthritis.

Zu etwa 500g auf etwa 85°C erwärmtes Wasser werden etwa 300g Polysorbat 80 von etwa 85°C zugegeben und die Mischung wird bei etwa 85°C so lange (etwa 5 Minuten) gerührt, bis die Mischung homogen und transparent geworden ist. Anschließend werden dieser Mischung etwa 200g reines Chondroitinsulfat hinzugegeben. Diese Mischung wird bei der genannten Temperatur wiederum so lange kräftig gerührt, bis sich ein homogenes und transparentes Zwischenprodukt ergeben hat. Nach Abkühlung auf Raum- oder Körpertemperatur bleiben Klarheit und Wasserlöslichkeit der so erhaltenen Phase mit einem Gehalt von etwa 20% Chondroitinsulfat erhalten.

### Beispiel 8: α-Liponsäure

Etwa 870 Gramm Polysorbat, vorzugsweise Polysorbat 80 werden auf etwa 120°C erhitzt und anschließend etwa 130 Gramm α-Liponsäure - zum Beispiel das Produkt alpha Liponsäure, Art.Nr. 1999/010, der Firma K.-W. Pfannenschmitdt GmbH, Hamburg - hinzugegeben und unter Beibehaltung der Temperatur etwa 10 Minuten gerührt, bis sich ein homogenes transparentes Gemisch ergeben hat.

Das auf diese Weise hergestellte Zwischenprodukt läßt sich nach Abkühlung in etwa 25°C warmem Wasser unter Rühren beliebig lösen. Klarheit und Wasserlöslichkeit der wasserfreien Phase bleiben auch dann erhalten, wenn ihrer wässrigen Lösung Magensäure (Salzsäure) zugegeben wird.

Aus Vorstehendem erkennt man, daß in Wasser nicht oder nur schwer lösliche physiologisch wirksame Wirkstoffe durch Behandlung mit einem körperverträglichen Lösungsvermittler mit einem HLB-Wert zwischen 9 und 16, vorzugsweise einem Polysorbat, insbesondere Polysorbat 80, in der Wärme und anschließende teilweise rasche Abkühlung auf Raumtemperatur eine in Wasser und Ölen lösliche Phase von niedriger Wirkstoffkonzentration erhalten werden kann, welche Micellen mit Radien zwischen etwa 10nm und etwa 20nm aufweist. Diese Phase ist gut beständig vor allem gegenüber Säuren wie etwa der Magensäure. Sie ist vor allem industriell einfacher zu handhaben als die Wirkstoffe selbst. Die Beständigkeit der Phase kann noch dadurch erhöht werden, daß man einen Hilfsstoff wie etwa Linolsäure ergänzend einsetzt. Wie gezeigt wurde, umgeben sich die den Wirkstoff enthaltenden Micellen mit mehreren den Hilfsstoff enthaltenden Micellen, welche einen Schutz der Wirkstoff-Micellen bilden.

Die erfindungsgemäß gebildeten Micellen sind chemisch, mikrobiologisch, mechanisch und thermisch sehr stabil. Sie enthalten anteilig eine viel größere Menge an meist lipophilen Wirkstoffen als vergleichbare Liposomen. Die erfindungsgemäßen Wirkstoffkonzentrate bzw. die gebildeten Wirkstoffphasen lassen sich mit Vorteil Lebensmitteln, Nahrungergänzungsmitteln, Haut-, Haar- und Zahnpflegemitteln sowie kosmetischen oder pharmazeutischen Mitteln zusetzen. Die Wirkstoffkonzentrate sind absolut magensäurestabil. Durch die Micellenbildung sind die Wirkstoffe für den Organismus wesentlich schneller verfügbar als in Emulsionen verabreichte Wirkstoffe. Die Resorption im intestinalen Bereich macht eine Beteiligung der Gallensäure überflüssig.

## Patentansprüche

1. Verfahren zur Herstellung einer wasserlöslichen Phase eines in Wasser nicht oder nur schwer löslichen physiologisch wirksamen Wirkstoffes **dadurch gekennzeichnet dass** der eine ω-3-Fettsäure enthaltende Wirkstoff in der Wärme mit einem körperverträglichen Lösungsvermittler mit einem HLB-Wert zwischen 9 und 16 im Überschuss zur Bildung eines transparenten Zwischenproduktes verrührt wird und das Zwischenprodukt auf Raumtemperatur abgekühlt wird.

2. Verfahren nach Anspruch 1, **dadurch gekennzeichnet, dass** ein Polysorbat, insbesondere Polysorbat 80 eingesetzt wird.

3. Verfahren nach Anspruch 1 oder 2, **dadurch gekennzeichnet, dass** dem Zwischenprodukt in der Wärme Wasser zugesetzt wird.

4. Verfahren nach einem der vorstehenden Ansprüche, **dadurch gekennzeichnet, dass** der Mischung aus Wirkstoff und Lösungsvermittler in der Wärme ein Hilfsstoff beispielsweise Linolsäure zugesetzt wird.

5. Verfahren nach Anspruch 4, **dadurch gekennzeichnet, dass** als Hilfsstoff Linolsäure eingesetzt wird.

6. Verfahren nach einem der vorstehenden Ansprüche, **dadurch gekennzeichnet, dass** der Lösungsvermittler in solchem Überschuss zugesetzt wird, dass das Zwischenprodukt eine Wirkstoffkonzentration von etwa 20% oder darunter aufweist.

7. Verfahren nach einem der vorstehenden Ansprüche, **dadurch gekennzeichnet, dass** der Lösungsvermittler auf eine Temperatur von etwa 80°C bis etwa 100°C erwärmt wird.

8. Verfahren nach einem der vorstehenden Ansprüche, **dadurch gekennzeichnet, dass** das Zwischenprodukt rasch abgekühlt wird.

9. Wasserlösliches Konzentrat eines in Wasser nicht oder nur schwer löslichen physiologischen, eine ω-3-Fettsäure enthaltenden Wirkstoffes, das einen körperverträglichen Lösungsvermittler mit einem HLB-Wert von 9 bis 16 im Überschuss aufweist.

10. Konzentrat nach Anspruch 9, **dadurch gekennzeichnet, dass** der Lösungsvermittler ein Polysorbat, insbesondere Polysorbat 80 ist.

11. Konzentrat nach Anspruch 9 oder 10, **dadurch gekennzeichnet, dass** es einen Hilfsstoff, wie beispielsweise Linolsäure aufweist.

12. Konzentrat nach einem der Ansprüche 9 bis 11, **dadurch gekennzeichnet, dass** es Wirkstoffinicellen mit einer durchschnittlichen Radiusverteilung von etwa 10nm bis etwa 20nm aufweist.

13. Konzentrat nach Anspruch 12, **dadurch gekennzeichnet, dass** es Hilfsstoffmicellen aufweist, die die Wirkstoffmicellen umlagern.

14. Verwendung des Konzentrats nach einem der Ansprüche 10 bis 13 als Zusatz zu Lebensmitteln, Nahrungsergänzungsmitteln, zu Haut- Haar- und Zahnpflegemitteln, sowie zu kosmetischen und/oder pharmazeutischen Mitteln.

15. Konzentrat nach einem der Ansprüche 9 bis 14 **gekennzeichnet durch** einen Wirkstoffgehalt von etwa 20% oder weniger.
